# EUROPEAN PATENT SPECIFICATION

(11) **EP 4 318 500 B1**
(45) Date of publication and mention of the grant of the patent: **25.11.2026**
(21) Application number: 22779064.9
(22) Date of filing: 31.03.2022
(51) Int. Cl.: G21K 5/08, A61N 5/10, G21K 1/00

(54) **NEUTRON CAPTURE THERAPY SYSTEM AND NEUTRON GENERATION PART RECOVERY METHOD THEREFOR**
NEUTRONENEINFANGTHERAPIESYSTEM UND VERFAHREN ZUR WIEDERHERSTELLUNG EINES NEUTRONENERZEUGUNGSTEILS DAFÜR
SYSTÈME DE THÉRAPIE PAR CAPTURE DE NEUTRONS ET SON PROCÉDÉ DE RÉCUPÉRATION DE PARTIE DE GÉNÉRATION DE NEUTRONS

(30) Priority: 31.03.2021 CN 202110349074
(43) Date of publication of application: 07.02.2024
(73) Proprietor: Neuboron Therapy System Ltd., Xiamen, Fujian 361026 (CN)
(72) Inventor: GONG, Qiu-ping, Nanjing, Jiangsu 211112 (CN)
(74) Representative: Gunzelmann, Rainer
(86) International application number: PCT/CN2022/084324
(87) International publication number: WO 2022/206883

(56) References cited:
- EP-A1- 4 122 534
- EP-A1- 4 324 516
- WO-A1-2021/185277
- WO-A1-2022/010795
- WO-A1-2023/045904
- CN-A- 101 521 981
- CN-A- 104 149 067
- CN-A- 109 925 607
- CN-A- 110 849 684
- CN-A- 111 686 376
- CN-U- 207 243 428
- CN-U- 207 264 793
- JP-A- 2013 019 692
- JP-A- 2014 236 913
- JP-A- 2019 215 294
- US-A1- 2020 246 639

## Description

### TECHNICAL FIELD

An aspect of the invention relates to a neutron capture therapy system; and another aspect of the invention relates to a method for recovering a neutron generation part of a neutron capture therapy system.

### BACKGROUND

With the development of atomics, radioactive ray therapy, such as cobalt sixty, a linear accelerator, an electron beam, or the like, has become one of the major means to treat cancers. However, traditional photon or electron therapy is restricted by physical conditions of radioactive rays themselves, and thus will also harm a large number of normal tissues on a beam path while killing tumor cells. Furthermore, owing to different levels of sensitivity of tumor cells to radioactive rays, traditional radiotherapy usually has poor treatment effect on malignant tumors (for example, glioblastoma multiforme and melanoma) with radio resistance.

In order to reduce radiation injury to normal tissues around tumors, a target therapy concept in chemotherapy is applied to radioactive ray therapy. With respect to tumor cells with high radio resistance, irradiation sources with high relative biological effectiveness (RBE), such as proton therapy, heavy particle therapy, neutron capture therapy, or the like, are also developed actively now. Here neutron capture therapy combines the abovementioned two concepts, for example boron neutron capture therapy (BNCT), provides a better cancer treatment choice than traditional radioactive rays, by specific aggregation of boron-containing drugs in tumor cells in combination with precise neutron beam regulation and control.

In a neutron capture therapy system with an accelerator, a charged particle beam is accelerated by the accelerator, to reach an energy sufficient to overcome Coulomb repulsive forces of atomic nucleus of a neutron generation part in a beam shaping body, and generate a nuclear reaction with the neutron generation part to generate neutrons. Therefore, during generation of the neutrons, the neutron generation part is subjected to irradiation of the accelerated charged particle beam with high-power, and temperature of the neutron generation part increases significantly, thereby affecting service life of the neutron generation part. In order to generate neutrons stably, it is necessary to recover and maintain an old neutron generation part and replace it by a new neutron generation part, therefore, it is necessary to periodically replace the neutron generation part. The neutron generation part irradiated by the accelerated charged particle beam with a high-energy-grade holds a large number of radioactive rays inevitably, so that when the neutron generation part is replaced at a short distance, there will be a hidden trouble of radiation safety inevitably without enough protection or isolation measures. A recovery of a neutron generation part is discussed e.g. in publication US 2020/246639 A1.

### SUMMARY

The invention is defined by the claims.

According to an aspect of the invention, there is provided a neutron capture therapy system according to claim 1. The neutron capture therapy system includes an accelerator configured to generate a charged particle beam, a transmission device configured to transmit the charged particle beam and including at least a first transmission part and a second transmission part, a neutron generation part made of radioactive consumables and configured to generate a neutron beam by reacting with the charged particle beam, a beam shaping body configured to perform spectrum adjustment on the neutron beam, and a guidance device. Here the first transmission part is detachably connected to the second transmission part and includes a first position and a second position, the neutron generation part is arranged at an end of the first transmission part, and the guidance device is capable of guiding the first transmission part to move from the first position to the second position. Here when the first transmission part is located at the first position, the neutron generation part at the end of the first transmission part is accommodated in the beam shaping body and is capable of reacting with the charged particle beam to generate neutrons; and when the first transmission part is located at the second position, the neutron generation part is separated from the beam shaping body, and at this time, each of the first transmission part and the neutron generation part are in a replaceable state.

When the neutron generation part made of radioactive consumables is located in the beam shaping body, a radiation shield in the beam shaping body may shield radioactive rays leaked from the neutron generation part to prevent the radioactive rays from irradiating an operator. When the neutron generation part is separated from the beam shaping body, the neutron generation part is exposed in an operation space, and then operation time of the operator may be shortened, and/or an operation distance between the operator and the neutron generation part may be increased, so that radiation leaked from the neutron generation part is prevented from irradiating the operator as much as possible in a process of the neutron generation part moving from the first position to the second position along with the first transmission part. According to the technical solution provided in this aspect of the invention, the guidance device may quickly, stably and reliably guide the neutron generation part to be recovered from the first operation position to the radiation shielding device at the second position, and in an operation process of replacing the neutron generation part, the operator has an operation condition of keeping an enough safe distance from the neutron generation part and even completely isolating from the neutron generation part, thereby avoiding excessive radiation damage effectively.

The guidance device includes a first guidance part and a second guidance part, of which guiding directions are different from each other. According to an embodiment of the invention, in case that available space in an operation chamber is narrow and limited, the first guidance part of the guidance device guides the first transmission part of the transmission device to move in a first direction along which the transmission device is arranged and then enter the second guidance part of the guidance device, to avoid some facilities and obstacles that may exist in the first direction. Such arrangement facilitates miniaturization and compactness of the operation chamber, improves utilization of the space, and reduces occupied area and construction cost.

Preferably, in a plane where the first guidance part is located parallel to the ground, a projection of a connection line between a first end and a second end of the second guidance part may form a first angle with the first guidance part; and in a plane where the first guidance part is located perpendicular to the ground, a projection of the connection line between the first end and the second end of the second guidance part may form a second angle with the first guidance part.

Preferably, the second guidance part may include at least two segments of guide rails, one of which is a fixed rail detachably connected to the first guidance part, and the other of which is movable rail rotatably connected to the fixed rail.

Preferably, the movable rail may be connected to the fixed rail through a hinge structure and may have two positions, and when the movable rail is located at a first position, the movable rail and the fixed rail keep a normal guidance state together; and when the movable rail is stressed and then located at a second position, the movable rail moves at a certain angle relative to the fixed rail, and then may automatically return to the first position.

Preferably, 90% (weight percentage) or more of materials of the guidance device may be composed of at least one of C, H, O, N, Si, Al, Mg, Li, B, Mn, Cu, Zn, S, Ca, or Ti.

Preferably, the first guidance part may be made of an aluminum alloy, a magnesium-aluminum alloy, a carbon fiber composite material, a glass fiber composite material, or a combination thereof.

Preferably, the second guidance part may select materials the same as the first guidance part, or may select materials different from the first guidance part, such as stainless steel and a ferrous material.

Preferably, a surface of the second guidance part may be coated with an anti-radiation film, or a removable anti-radiation protective cover may be added to the surface of the second guidance part.

Preferably, a shaped surface of the anti-radiation protective cover may be fitted with a cross-sectional shape of the second guidance part.

The neutron capture therapy system further includes a movable radiation shielding device configured to accommodate the first transmission part and the neutron generation part guided by the guidance device, and the radiation shielding device recovers the first transmission part moving along the guidance device and the neutron generation part at an end thereof together.

The guidance device includes a first guidance part and a second guidance part, of which guiding directions are different from each other.

The guidance device further includes a third guidance part which is fixedly accommodated in the movable radiation shielding device.

Preferably, the second guidance part is detachably connected to the third guidance part, or the second guidance part may be flexibly aligned with and connected to the third guidance part, to conveniently guide the first transmission part of the transmission device into the radiation shielding device.

The radiation shielding device is made of a radiation shielding material and includes an openable member, the radiation shielding device forms an accommodation opening when the openable member is opened, the first transmission part of the transmission device enters the radiation shielding device from the accommodation opening; and the radiation shielding device forms a closed shielding space when the openable member is closed, to prevent radiation leakage of the neutron generation part.

The movable radiation shielding device further includes a buffer device capable of generating elastic deformation after the first transmission part is in contact with the buffer device, generating elastic deformation and applying an opposite acting force in a movement direction of the first transmission part, so that the first transmission part is safely stopped in the radiation shielding device, to avoid damage to at least the first transmission part or the radiation shielding device. Furthermore, the neutron capture therapy system includes a positioning system enabling the radiation shielding device to move to a preset position, so as to be docked with the guidance device. The positioning system may mark the position in advance according to means such as structural positioning, three-dimensional (3D) algorithm positioning, or the like, so that the radiation shielding device may move to an ideal position aligned with the guidance device, thus the first transmission part and the neutron generation part at the end thereof may be reliably recovered to the radiation shielding device after moving to a tail end of the guidance device.

Preferably, the movable radiation shielding device may further include a positioning system enabling the radiation shielding device to move to a preset position, so that the second guidance part is docked with the third guidance part.

The neutron generation part enters the accommodation opening of the movable radiation shielding device along the guidance device, later than the first transmission part, and the openable member is closed after the neutron generation part completely enters the movable radiation shielding device.

Preferably, after the openable member of the movable radiation shielding device is closed, an internal vacuumized environment or inert gas inflation may be implemented, while physical isolation from external air is implemented.

Preferably, the neutron capture therapy system may further include a control part capable of implementing opening and closing of the openable member of the movable radiation shielding device through remote control via wired or wireless connection.

The neutron capture therapy system further includes a driving device providing power to enable the first transmission part to be separated from the beam shaping body, move out of the first position and move along the guidance device.

Preferably, the neutron capture therapy system may further include a support device stably supporting the first transmission part in a direction along which the transmission device is arranged.

Preferably, the guidance device may include a first guidance part and a second guidance part, of which guiding directions are different from each other.

Preferably, the driving device may include a driving frame carrying the first transmission part and the neutron generation part, and a power structure providing a driving force.

Preferably, the neutron capture therapy system may further include a control part which may implement controlling the power structure of the driving device through remote control via wired or wireless connection, to provide power for movement of the first transmission part and the neutron generation part between the first position and the second position.

Preferably, the driving frame of the driving device may include at least one set of roller structures.

Preferably, the driving frame of the driving device may include at least two sets of roller structures, and rolling directions of the two sets of rollers are different.

Preferably, rolling planes of the two sets of rollers of the driving frame are different and perpendicular to each other.

Preferably, the at least two sets of roller structures of the driving frame are combined with concave and/or convex sliding rails of the guidance device, to implement transmission movement, and rolling transmission may be implemented by selecting a structure where one or two sets of rollers are matched with the concave and/or convex sliding rails.

Furthermore, the neutron capture therapy system may further include a cooling device configured to cool the neutron generation part and including a cooling pipeline, when the first transmission part is located at the first position, the cooling pipeline is in an on state, and the cooling device performs cooling operation; and when the first transmission part is located at the second position, the cooling pipeline is in an off state, the cooling device cannot perform normal operation, and the cooling device and the first transmission part are recovered and replaced together.

Preferably, the neutron capture therapy system may further include a detection device including a detection circuit, and when the first transmission part is located at the first position, the detection device is in an on state to perform detection operation; and when the first transmission part is located at the second position, the detection circuit is in an off state and cannot perform normal operation, and the detection device is recovered and replaced together with the cooling device, the neutron generation part and the first transmission part. The "detection device" described here may be a charged particle sensor, or may be a device adapted to be installed at the first transmission part, such as a vacuum pressure sensor, and/or a neutron detector, or the like.

According to another aspect of the invention, there is provided a method according to claim 6 for recovering the neutron generation part of the neutron capture therapy system as described above, the neutron capture therapy system further includes an auxiliary pipeline connected to the neutron generation part or the first transmission part, the method for recovering the neutron generation part includes the following operations. The first transmission part and the second transmission part are split apart. The auxiliary pipeline is disconnected from the neutron generation part. A first power is applied to the first transmission part arranged with the neutron generation part, to separate the first transmission part from the beam shaping body along the guidance device and from the first position. A second power is applied to the first transmission part arranged with the neutron generation part, to move the first transmission part along the guidance device again, until to a recovery position.

The neutron capture therapy system further includes a shielding facility provided with an opening, and the first transmission part passes through the opening in a state where the shielding facility is kept closed.

Furthermore, the method for recovering the neutron generation part may be carried out through the opening in the state where the shielding facility is kept closed.

Furthermore, the shielding facility may be provided with an auxiliary door, the auxiliary door at least partially shields the opening of the shielding facility and has an opening matched with contours of the first transmission part, the guidance device and the auxiliary pipeline arranged at the opening of the shielding facility when the auxiliary door is closed, and the method for recovering the neutron generation part may be carried out through the opening in a state where the shielding facility is kept closed and the auxiliary door is kept open.

The neutron capture therapy system further includes the radiation shielding device configured to accommodate the neutron generation part after recovery, and a first space and a second space separated by the shielding facility, an anti-radiation protective cover is arranged on the guidance device, the neutron generation part is located in the first space, the second transmission part is located in the second space, and the method for recovering the neutron generation part further includes the following operations. Before applying the first power to the first transmission part arranged with the neutron generation part, the radiation shielding device is moved to the second space and the radiation shielding device is fixed at a preset position, the openable member of the radiation shielding device is opened, and the anti-radiation protective cover on the guidance device is removed. After applying the second power to the first transmission part arranged with the neutron generation part, the first transmission part is moved into the radiation shielding device along the guidance device, the openable member of the radiation shielding device is closed, the anti-radiation protective cover is covered on the guidance device, and the radiation shielding device is pushed out of the second space.

Further, there is provided a method for recovering the neutron generation part of the neutron capture therapy system as described above, the neutron capture therapy system further includes a shielding facility keeping closed during recovery operation and provided with an opening, and the method for recovering the neutron generation part includes the following operations. A cooling pipeline connected to the neutron generation part is disconnected through the opening of the shielding facility. A first power is applied to the first transmission part arranged with the neutron generation part, to separate the first transmission part from the beam shaping body along the guidance device and from the first position, and move the first transmission part to a position close to the opening of the shielding facility. Then, a second power is applied to the first transmission part arranged with the neutron generation part, to move the first transmission part along the guidance device again, until to a recovery position. Preferably, the method for recovering the neutron generation part may further include the following operations. A detection circuit of a detection device and other connection lines are cut off while the cooling pipeline is cut off. When the neutron generation part is separated from the beam shaping body, exposure of the neutron generation part may bring large radiation, the shielding facility is kept closed during recovery, and the operator performs replacement operation of the neutron generation part through the opening in the shielding facility only, so that radiation leakage of operation environment is greatly reduced, and operation safety is improved.

Further, there is provided a method for recovering the neutron generation part of the neutron capture therapy system as described above, including the following operations. A shielding facility is kept closed during recovery operation, and the shielding facility is provided with an auxiliary door which is kept in a closing state during system operation. When the system stops and the neutron generation part needs to be replaced, the auxiliary door is opened, a cooling pipeline connected to the neutron generation part is disconnected through an opening of the auxiliary door. A first power is applied to the first transmission part arranged with the neutron generation part, to separate the first transmission part from the beam shaping body along the guidance device and from the first position, and move the first transmission part to a position close to the opening of the shielding facility. Then, a second power is applied to the first transmission part arranged with the neutron generation part, to move the first transmission part along the guidance device again, until the first transmission part enters a recovery position in a radiation shielding device, and the auxiliary door is closed. Preferably, shape of the opening in the auxiliary door is usually fitted with shapes of the first transmission part, the guidance device and mating pipeline structures, a vacuum valve, or the like arranged at the opening, and the shape includes, but is not limited to, oval shape, rhombus shape, square shape, even an irregular shape, or the like.

In the embodiment of the invention, time for the operator to be exposed in an environment of a strong radiation object in a process of replacing radioactive consumables is shortened, and a relatively safe operation distance is provided for the operator and the neutron generation part, so that in an operation process of replacing the radioactive consumables, the operator may have an operation condition of keeping an enough safe distance from the radioactive consumables and even completely isolating from the radioactive consumables, so that contact between the operator and radioactive rays is reduced, and a hidden trouble of radiation safety is reduced. Furthermore, in the embodiment of the invention, the neutron capture therapy system belonging to a radioactive ray irradiation system is compact in structure and convenient in installation, and in a condition where operation space is limited, actual space and place are efficiently utilized, occupied area and construction cost are reduced, thus a reliable, stable and safe recovery solution is provided for the neutron generation part.

### BRIEF DESCRIPTION OF THE DRAWINGS

FIG. 1 is a schematic plan view of a neutron capture therapy system according to an embodiment of the invention.
FIG. 2 is a schematic perspective view of a neutron capture therapy system according to an embodiment of the invention, showing a first transmission part of a transmission device at a first position and a radiation shielding device in a closed state.
FIG. 3 is a schematic perspective view of a neutron capture therapy system according to an embodiment of the invention, showing a first transmission part of a transmission device at a second position and a radiation shielding device in an open state.
FIG. 4 is a schematic top view of a guidance device of a neutron capture therapy system according to the embodiment of FIG. 1.
FIG. 5 is a schematic plan view of a neutron capture therapy system according to another embodiment of the invention.
FIG. 6 is a schematic perspective view of a guidance device of a neutron capture therapy system according to another embodiment of FIG. 5.
FIG. 7 shows a schematic plan view of a first transmission part of a transmission device.
FIG. 8 is a schematic cross-sectional view of a roller set of a driving frame of a driving device of a neutron capture therapy system.
FIG. 9A and FIG. 9B are schematic usage view and schematic cross-sectional view of an anti-radiation protective cover of a guidance device of a neutron capture therapy system.
FIG. 10 is a schematic perspective view of a neutron capture therapy system according to an embodiment of the invention, showing a schematic view of recovering a first transmission part and a neutron generation part when a shielding facility is in a closed state.
FIG. 11A to FIG. 11E are schematic views of an auxiliary door of a radiation shielding device of a neutron capture therapy system, and opening and closing states thereof.

### DETAILED DESCRIPTION

Application of neutron capture therapy as an effective means for cancer treatment gradually increases in recent years, in which boron neutron capture therapy (BNCT) is most commonly seen, and neutrons supplied to BNCT may be supplied by a nuclear reactor or an accelerator. Embodiments of the invention use an accelerator BNCT as an example, and basic components of the accelerator BNCT usually includes an accelerator configured to accelerate charged particles (such as protons, deuterium cores, or the like), a neutron generation part made of radioactive consumables, a thermal removal system, and a beam shaping body. Here the accelerated charged particles act with the neutron generation part made of metal to generate neutrons, and an appropriate nuclear reaction may be selected according to characteristics such as a desired neutron yield and energy, available energies of the accelerated charged particles, a current, physical and chemical properties of the neutron generation part made of metal, or the like. Nuclear reactions as commonly discussed include ⁷Li(p, n)⁷Be and ⁹Be(p, n) ⁹B, both of which are endothermic reactions and have energy thresholds of 1.881 MeV and 2.055 MeV respectively. An ideal neutron source for BNCT is an epithermal neutron at a keV energy level, then theoretically, when protons with energies only slightly higher than the threshold are used to bombard the neutron generation part made of metallic lithium, neutrons with relatively low energies may be generated for clinical application without too much retarding treatment. However, proton action sections of two neutron generation parts made of metallic lithium (Li) and beryllium (Be) with the threshold energy are not high, therefore protons with higher energies are usually selected to initiate a nuclear reaction, to generate a large enough neutron flux.

An ideal neutron generation part made of radioactive consumables should have a high neutron yield, the generated neutron energy distribution is close to an epithermal neutron energy region (it will be described in detail below), there is not too much strong penetrating radiation, and there are characteristics such as safe, cheap, easy to operate, resistant to high temperature, or the like. However, nuclear reactions that meet all requirements may not be found actually, and the neutron generation part made of metal Li is used in the embodiments of the invention. However, it is well known by those skilled in the art that the neutron generation part may also be made of another metallic material other than metallic materials as mentioned above.

Requirements of the thermal removal system vary depending on the selected nuclear reaction. For example, requirements of the thermal removal system are higher in ⁷Li(p, n) ⁷Be than in ⁹Be(p, n) ⁹B, due to poor melting point and thermal conductivity of the neutron generation unit made of metal (metal Li). ⁷Li(p, n) ⁷Be nuclear reaction is used in the embodiments of the invention. It may be known therefrom that temperature of the neutron generation part irradiated by an accelerated charged particle beam with a high energy level inevitably increases greatly, thereby affecting service life of the neutron generation part.

Therefore, the neutron capture therapy system belonging to a radioactive ray irradiation system inevitably has a problem of replacing the neutron generation part made of radioactive consumables. In order to solve the problem while contact between the operator and radioactive rays is reduced as much as possible, embodiments of the invention provide a neutron capture therapy system, which may stably and reliably recover the neutron generation part, have a limited operation space in a direction of the transmission device and a vertical direction of the neutron generation part, utilize the space effectively, avoid interference and obstruction of operation spaces at the same horizontal height in the direction of the transmission device, remove an acceleration device for protons in the transmission device, reduce configuration of the operation space, and reduce occupied area and construction cost as a whole.

In order to solve the problem of replacing the neutron generation part made of radioactive consumables while contact between the operator and radioactive rays is reduced as much as possible, embodiments of the invention provide a neutron capture therapy system.

Because main radiation to the operator comes from radioactive rays generated by a nuclear reaction occurred after the charged particle beam is irradiated to the neutron generation part made of radioactive consumables, the embodiments of the invention are intended to describe disassembling the neutron generation part which reaches its service life and is required to be recovered after occurrence of the nuclear reaction, and do not relate to installation of a new neutron generation part. Furthermore, orientation words such as upper, lower, horizontal, vertical, or the like mentioned in the embodiments of the invention are used to facilitate descriptions and describe positional relationships between components according to illustrated directions, and are not intended to limit substantial absolute directions thereof.

Preferred implementations of the neutron capture therapy system are described below with reference to the drawings.

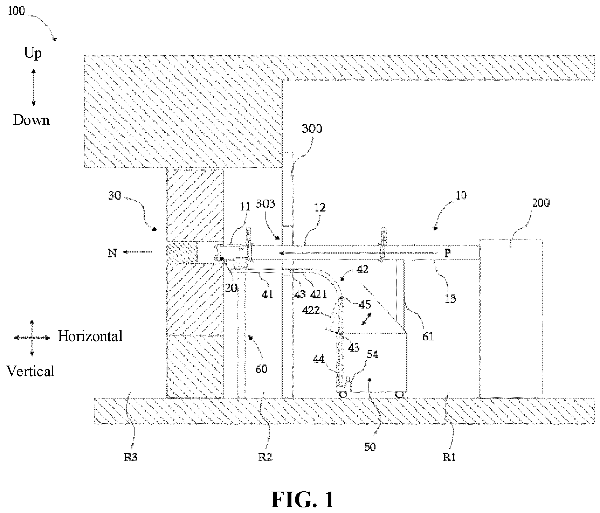

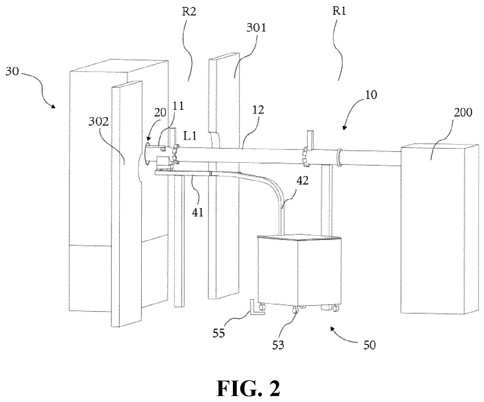

FIG. 1 and FIG. 2 are schematic plan view and schematic perspective view of a neutron capture therapy system according to an embodiment of the invention, respectively. As shown in FIG. 1 and FIG. 2, the neutron capture therapy system 100 includes a transmission device 10, a neutron generation part 20 containing radioactive consumables, a beam shaping body 30, a guidance device 40, a radiation shielding device 50, and a support device 60. Here the neutron capture therapy system 100 includes at least a first space R1 and a second space R2, and the first space R1 includes an accelerator 200 configured to generate a charged particle beam P, a part of the transmission device 10 configured to transmit the charged particle beam P, a support device configured to support the transmission device, a detection device, or the like. A transmission direction of the charged particle beam P is consistent with an arrangement direction of the transmission device 10, and the transmission device 10 penetrates the first space R1 and the second space R2. The second space R2 is a core reaction space of the whole system, has a relatively large radiation amount, and is physically separated from the first space R1 through a shielding facility 300, so that radiation amount in the first space R1 is within a relatively safe and controllable range. The shielding facility 300 includes at least a shielding door 301 and/or 302, and further includes at least one opening 303 to allow the transmission device 10 to pass there-through when the shielding facility 300 is in a closed state. In the illustrated embodiment, interior of the transmission device 10 is in a vacuum state and includes at least a first transmission part 11 and a second transmission part 12 which are detachably connected to each other. Shape of each of the first transmission part and the second transmission part is preferably a hollow tubular shape, and cross-sectional shape of each of the first transmission part and the second transmission part includes, but is not limited to, circular shape, oval shape, diamond shape, square shape, irregular shape, or the like. Shape of at least one opening of the shielding facility 300 may be fitted with shape of the transmission part passing through the opening, including but not limited to circular shape, oval shape, diamond shape, square shape, irregular shape, or the like. In a process of replacing radioactive consumables and splitting the transmission device, each of the first transmission part and the second transmission part of the transmission device may preferably maintain a vacuum state. The second space R2 includes the first transmission part 11 of the transmission device 10, the neutron generation part 20 arranged at an end portion of the transmission device 10 and reacting with the charged particle beam P to generate a neutron beam N, and the beam shaping body 30 configured to perform spectrum adjustment on the neutron beam N. The transmission device 10 transmits charged particles P accelerated by the accelerator 200 from the first space R1 to the neutron generation part 20 in the second space R2. The accelerator 200 accelerates the charged particles P to an energy sufficient to overcome an atomic nuclear force of the target, and produces ⁷Li(p, n) ⁷Be nuclear reaction with the neutron generation part 20 to generate neutrons which form the neutron beam N emitted from a beam outlet. The beam shaping body 30 is usually large in volume and is not suitable for movement, and is fixedly arranged inside a wall body in an embedded manner. The beam shaping body 30 includes a retarder, a thermal neutron absorber, a radiation shield, or the like (not shown in the drawings). Main material selected by the retarder is usually aluminum fluoride, and optionally, a mixed material of one or more of lithium fluoride, aluminum, lead fluoride, aluminum oxide, calcium fluoride, or magnesium fluoride. Some of these materials have high brittleness, and thus require high requirements of processing and installation processes. They slow down neutrons generated by the neutron generation part 20 to the epithermal neutron energy zone, and deviated neutrons are guided back to the retarder by a reflector to improve intensity of an epithermal neutron beam. The thermal neutron absorber absorbs thermal neutrons, to avoid unnecessary damage to normal tissues at shallow layers due to excessive dose during treatment. The radiation shield is configured to shield leaked neutrons and photons to reduce dose of normal tissues at non-irradiation regions. The neutron capture therapy system 100 further includes a third space R3 as an irradiation chamber, a neutron beam meeting a condition may be emitted from the beam outlet and enter the irradiation chamber for its usage.

FIG. 2 shows a state of opening the shielding facility 300 between the first space R1 and the second space R2, and when the shielding door 301 and/or the shielding door 302 are opened, the first space R1 is communicated with the second space R2. With reference to FIG. 1, the neutron capture therapy system 100 includes a support device 60 and/or 61 configured to stably support the transmission device 10 in a direction along which the transmission device is arranged. The support device 60 is arranged at a lower portion of the first transmission part 11, an end of the first transmission part 11 arranged with the neutron generation part 10 is accommodated in the beam shaping body 30, the first transmission part 11 is located at a first position L1. In an alternative embodiment, the support device 60 may also be arranged at different orientations such as upper and side portions of the first transmission part 11, or the like, to support it.

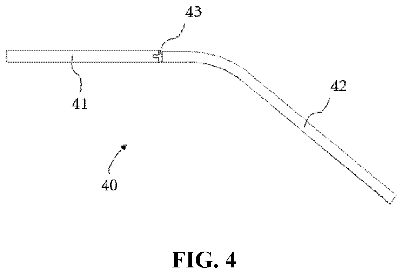

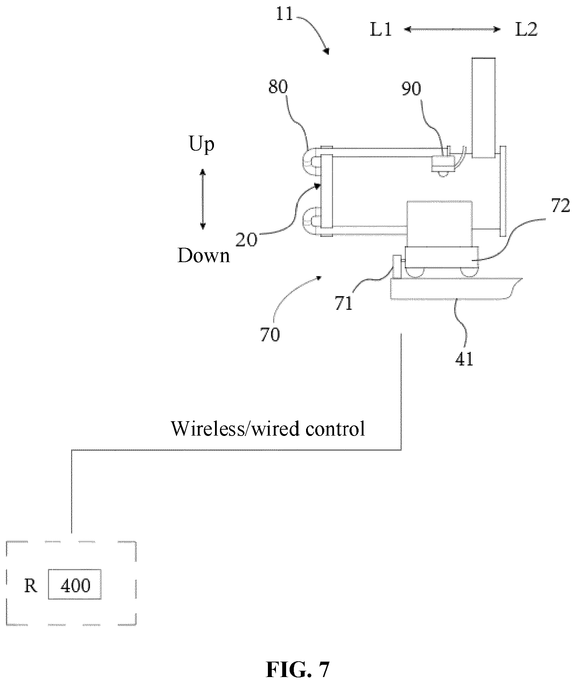

With reference to FIG. 1 and FIG. 2, the guidance device 40 is arranged between the support device 60 and the first transmission part 11 (referring to FIG. 4), the neutron capture therapy system 100 further includes a driving device 70 (referring to FIG. 7), the guidance device 40 may include a first rail (a first guidance part) 41 and a second rail (a second guidance part) 42. The first rail 41 substantially extends in a first direction along which the transmission device is arranged, and the first direction is opposite or identical to a movement direction of the charged particle beam P. A first end of the second rail 42 is connected to the first rail 41 through a connection part 43, a second end of the second rail 42 extends in a second direction which is different from the first direction, that is, the guidance device 40 has at least two guiding directions. Guiding directions of the second rail 42 and the first rail 41 are different from each other, which facilitates application of such arrangement in a compact building. With reference to FIG. 4, in an embodiment, in a plane where the first rail is located parallel to the ground, a projection of a connection line between the first end and the second end of the second rail 42 forms a first angle with the first rail 41, for example, the first angle ranges from 10° to 150°, preferably 30° to 90°; and in a plane where the first rail is located perpendicular to the ground, a projection of the connection line between the first end and the second end of the second rail 42 forms a second angle with the first rail 41. Extension direction of the second rail 42 may avoid other structures, such as the second transmission part 12, and/or the support device 61 of the third transmission part 13, or a beam splitting device, or the like; and in case that the first space R1 has limited operation space in a horizontal direction along which the transmission device 10 is arranged and a vertical direction perpendicular to the ground, a limited operable space is efficiently utilized. In an alternative embodiment, the second rail 42 may also be located in a plane where the first rail 41 is located perpendicular to the ground. The first rail 41 and the second rail 42 are detachably connected to each other through the connection part 43, or there may be a structure where the first rail 41 and the second rail 42 are integrally formed with each other. When the first rail 41 and the second rail 42 form a detachable connection structure, the connection part 43 may be a connection rod, a locking buckle, a concave & convex buckle, or another structure, which may firmly clamp the first rail 41 to the second rail 42, and enable the first transmission part 11 to smoothly slide from the first rail 41 to the second rail 42. An extension length of the first rail 41 limits a certain safety distance, allowing the operator to maintain beyond the safety distance to perform operations such as splitting, debugging, or the like. The second rail 42 is at least partially located in the first space R1, and preferably, the second rail 42 may further include at least two segments of rails 421 and 422. Here an end of the rail 421 is fixedly connected to the first rail 41 through the connection part 43, and the rail 422 is preferably hinged to another end of the rail 421 through a hinge structure 45 and has two positions. The first position is a position where a normal guidance state is kept with the fixed rail 421. The second position is a buffer position where an angle may be formed relative to the first position after subjected to a force during transmission. Then, the rail 422 may automatically rotate and return to the first position. These flexible rail segments are beneficial to improve flexibility and agility of the whole transmission device, and protect the whole system and devices from rigid damage during transmission operations, and are beneficial for subsequent alignment operations. A downward-extending end of the second rail 42 faces the radiation shielding device 50 below it, and the second rail 42 may not be connected to the radiation shielding device, or may at least partially keep connected to the radiation shielding device, and preferably, the movable rail 422 of the second rail 42 is rotatably aligned with the radiation shielding device. Neutrons are generated in the beam shaping body 30, materials around the beam shaping body 30 are activated most seriously, and the guidance device is partially arranged in the core reaction space R2 with a relatively large radiation amount, therefore material of the guidance device should also be selected carefully. In order to avoid too fast deterioration caused by serious activation, elements having small sections acting with neutrons or short half-life periods (less than 1 year) of radioactive isotopes generated after it is activated by neutrons are usually selected to form the guidance device. For example, 90% (weight percentage) or more of materials of a portion, subjected to large radiation, of the guidance device are composed of at least one of C, H, O, N, Si, Al, Mg, Li, B, Mn, Cu, Zn, S, Ca, or Ti. In the embodiment, When the guidance material selects an aluminum alloy, the aluminum has a short half-life period of only 2.2 minutes after it is activated by neutrons; however, element such as iron, cobalt, nickel, or the like contained in a traditional steel material has a long half-life period after it is activated by neutrons, for example, the half-life period of cobalt 60 is 5.27 years. Usage of the aluminum alloy significantly reduces radioactivity derived from neutron activation within a limited period of time, which is more beneficial to disassemble devices in future, in addition to reasonably suppressing dose caused by secondary radiation. The material of the guidance device may further be an aluminum-magnesium alloy, or may be a carbon fiber composite material, a glass fiber composite material, or a combination thereof. The radiation shielding device 50 includes an openable shielding plate 51, an accommodation opening 52 formed by opening the openable shielding plate 51, a movement member 53 and a buffer device 54 inside the movement member 53. The radiation shielding device 50 is made of a radiation shielding material, including, but not limited to, a radiation shield made of lead (Pb), or the like. A wheel-type movement member 53 is arranged at bottom of the radiation shielding device 50, and the openable shielding plate 51 made of a shielding material is covered at top of the radiation shielding device 50, as an openable member. Opening and closing of the openable shielding plate include, but are not limited to, rotation, sliding, or other opening manners. When replacement operation of the neutron generation part is performed, the operator controls the radiation shielding device 50 to move from an external space into the first space R1, to be located at a preset position and docked with a lower end of the second rail 42. In the embodiment, a positioning member 55 is further arranged at the ground, and may stop and position the radiation shielding device 50. In an alternative embodiment, the movement member may be a pre-laid rail or trolley, or the like, and the positioning member may be one or more markers, stop blocks, recessed structures, or the like; the driving device may be a hook, a rope or a cylinder, a linear motor, a chain transmission structure, or the like, which is controlled at a certain spaced distance, and may also be controlled by a wired signal or a wireless signal; and the radiation shielding device 50 includes, but is not limited to, a cube, a sphere, or the like.

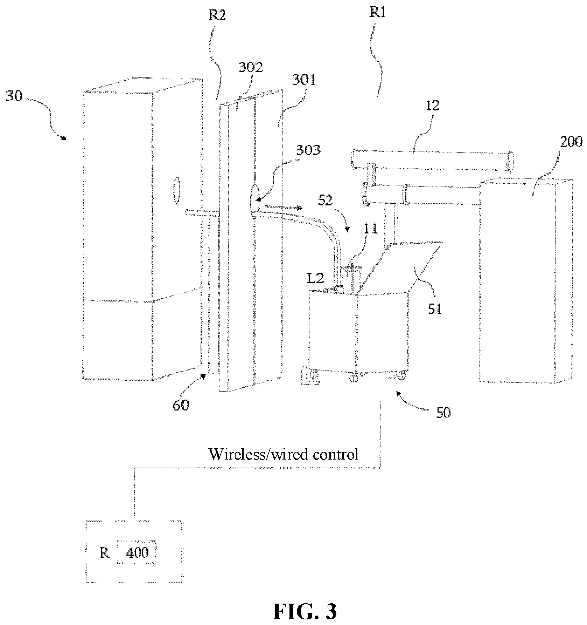

The embodiment of FIG. 3 shows a state where replacement operation is performed on the first transmission part 11 with the neutron generation part 20 at an end thereof, the shielding facility 300 is opened, the shielding door 301 and/or the shielding door 302 are opened, and at this time, the first space R1 is communicated with the second space R2. The operator manually or automatically opens the openable shielding plate 51 of the radiation shielding device 50, checks connection and position conditions of the first rail 41, the second rail 42 and the radiation shielding device 50, and performs the replacement operation. With reference to FIG. 1, the transmission device 10 further includes a third transmission part 13, the first transmission part 11 and the second transmission part 12 are split apart, the second transmission part 12 and the third transmission part 13 are split apart, and an overall length of the transmission device 10 is changed by moving the second transmission part 12 out, so that an avoidance space is left, to enable the first transmission part 11 with the neutron generation part 20 at the end thereof to move out along the rail. In splitting processes, the third transmission part may also preferably maintain a vacuum state. In an alternative embodiment, the second transmission part 12 may be fixedly connected to an end of the third transmission part 13, and may be designed as a structure such as a corrugated pipe or a telescopic pipe (not shown) to leave space for the replacement operation. Power from the driving device 70 (referring to FIG. 7) separates the first transmission part 11 with the neutron generation part 20 at the end thereof from the beam shaping body 30, the first transmission section 11 passes through the opening formed by opening the shielding door 301 and the shielding door 302 on the first rail 41 and in a direction along which the transmission device 10, and thus moves to the second rail 42. Under action of gravity or power of the rail, the first transmission part 11 continues to move along the direction of the second rail 42, so that the first transmission part 11 enters the accommodation opening 52 formed by opening the openable shielding plate 51, and at this time, the first transmission part 11 is located at a second position L2. Interior of the radiation shielding device 50 includes at least one buffer device 54 which may buffer the first transmission part 11 and the neutron generation part 20 sliding down. After the first transmission part 11 reaches cavity of the radiation shielding device 50 to be in contact with the buffer device 54, the buffer device generates elastic deformation, so that the first transmission part 11 and the neutron generation part 20 are statically accommodated in the radiation shielding device 50, and the first transmission part and the neutron generation part are prevented from being damaged due to their rapid collision with the radiation shielding device. In an alternative embodiment, when the second rail 42 of the neutron capture therapy system 100 has a short length, a third rail 44 may also be included, an end of the third rail 44 may also be connected to the second rail 42 through the connection part 43, and another end of the third rail 44 is installed inside the radiation shielding device 50. In a more preferred solution, the second rail 42 is hinged with a flexibly rotatable rail 422 structure, which facilitates linkage and alignment with the third rail 44 inside the radiation shielding device 50, without rigid connection with the connection part 43, thereby saving installation operations. A buffer cylinder 54 is arranged close to bottom of the third rail 44, and may generate deformation in movement directions of the first transmission part 11 and the neutron generation part 20 to apply opposite acting force, so as to stop and protect the first transmission part 11 and the radiation shielding device 50, and under action of the buffer cylinder 54, the first transmission part 11 and the neutron generation part 20 are safely stopped in the radiation shielding device 50. In an alternative embodiment, the buffer device may also be a different mechanical buffer structure, such as a spring or the like, or may be made of an elastic material, such as a rubber pad, an air bag, or the like. It should be noted that segments of the first transmission part 11 and the neutron generation part 20 preferentially entering the radiation shielding device 50 are sides opposite to the neutron generation part 20 containing radioactive consumables, that is, when recovery operation is completed finally, the neutron generation part 20 containing radioactive consumables is closer to the accommodation opening 52 of the radiation shielding device 50 and stays at a distal end of the buffer device. Design of such recovery direction focuses on improvement of cyclic utilization rate of the neutron generation part 20 containing radioactive consumables. The neutron generation part 20 located in the beam shaping body 30 is made of relatively active metallic lithium (Li) and/or beryllium (Be), and a coating film of the neutron generation part 20 has thickness of only about 100 microns and is relatively easily damaged after it is impacted. Therefore, a recovery direction along which the neutron generation part 20 is the last one to enter the accommodation opening and stays at the distal end of the buffer device is safer than other recovery directions, and the neutron generation part is not easily damaged, greatly improving reuse rate of the neutron generation part containing radioactive consumables and avoiding unnecessary radiation leakage after collision. Furthermore, direction of the neutron generation part 20 is unnecessary to be switched, a tedious and complex direction switching structure or the like are removed, more space is saved, and radiation leakage caused by fault of the switching structure or the like is also avoided. The interior of the radiation shielding device 50 may also implement a vacuumized environment or may be filled with an inert gas, to implement a function of physical isolation from external air, thereby improving safety and reliability of transporting and storing the first transmission part 11 and the neutron generation part 20 or other elements which are easy to have high reactivity.

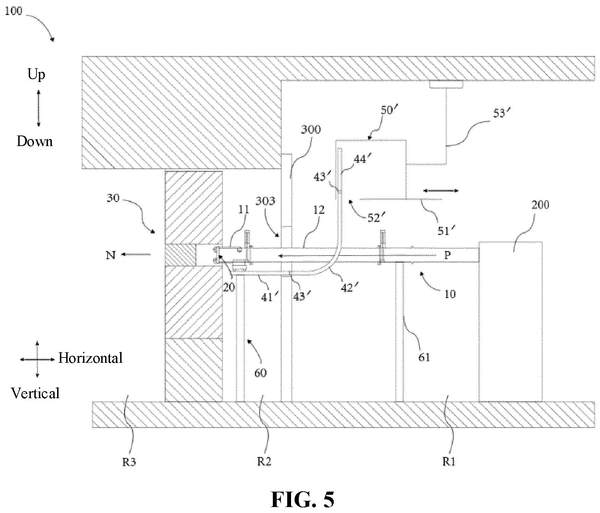

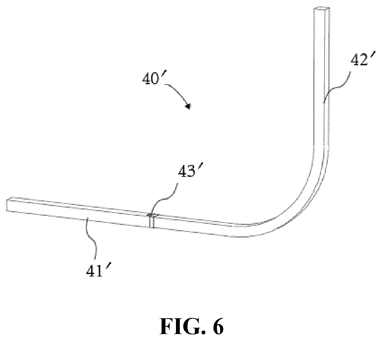

FIG. 5 and FIG. 6 show a guidance device 40' according to another embodiment of the invention. The guidance device 40' includes a first rail 41' and a second rail 42', the first rail 41' substantially extends in a first direction along which the transmission device is arranged, an end of the second rail 42' is connected to the first rail 41' through a connection part 43', and the second rail 42' defines a second direction extending upward. An operable mobile mechanical structure 53' is included at top of the first space R1, and supports a radiation shielding device 50' to implement spatial positioning through 3D calculation or manual adjustment, move to a preset 3D positioning marker and is connected to the second rail 42'. An openable shielding plate 51' is arranged at bottom of the radiation shielding device 50' and is operably opened to form an accommodation opening 52'. A third rail 44' is arranged at an inner side of the radiation shielding device 50' and is operably aligned with and connected to another end of the second rail 42' through the connection part 43'. The driving device 70 (referring to FIG. 7) transmits power through the rails, moves the first transmission part 11 and the neutron generation part 20 out from the first position L1, transmits the first transmission part 11 and the neutron generation part 20 along the first rail 41', through the second rail 42', to the third rail 44' in the radiation shielding device 50, and limits and fixes the first transmission part 11 and the neutron generation part 20 to reach a second position (not shown). The second rail 42' is operably disconnected from the connection part 43' at the third rail 44', and the openable shielding plate 51' is operably closed to complete recovery operation of the first transmission part 11 and the neutron generation part 20. In an alternative embodiment, the mobile mechanical structure 53' may also be located at a different position such as a side wall of the first space R1, or the like, and the second rail may be arranged in any other directions different from extension direction of the first rail (i.e., the direction along which the transmission device is arranged) according to an actual situation.

FIG. 7 shows a schematic view of the first transmission part 11 of the transmission device 10, the neutron capture therapy system 100 further includes a cooling device 80 configured to cool the neutron generation part 20 and a detection device 90, besides a driving structure 70. The driving structure 70 has power and further includes a driving frame 72 carrying the first transmission part 11 and the neutron generation part 20, and a power structure 71, and the power structure 71 includes, but is not limited to, an electronic or pneumatic connection rod, a robot and structures thereof, a mechanical arm, or the like. With reference to FIG. 1 and FIG. 2, the invention is intended to implement unmanned operation in case that the operator leaves the radiation exposure space R2 when the neutron generation part 20 is separated from the beam shaping body 30 and radiation dose is high in the second space R2 environment. Therefore, driving of the related power structure is a remote operation implemented by the operator operating a master control panel 400 in a non-radioactive safe area R. According to the remote operation implemented by the operator operating the master control panel 400 in the non-radioactive safe area R, the power structure 71 of the driving device 70 drives the driving frame 72 carrying the first transmission part 11 and the neutron generation part 20 to move, so that the driving frame 72 drives the first transmission part 11 and the neutron generation part 20 to be separated from the beam shaping body 30 and move along the guidance device; when the first transmission part 11 and the neutron generation part 20 move to the radiation shielding device 50, a pneumatic lever of the buffer device 54 is driven by the master control panel 400 to implement safe recovery of the first transmission part 11 and the neutron generation part 20, and then the master control panel 400 is operated so that the openable shielding plate 51 of the radiation shielding device 50 is closed, thereby completing recovery operation. With reference to FIG. 3, in an alternative embodiment, the shielding facility 300 and the auxiliary door on the shielding facility may also implement remote wired or wireless operation and control through the master control panel 400. The cooling device 80 includes a pipeline located at an end portion of the transmission device 10 and in planar contact with the neutron generation part 20, and two cooling pipelines arranged up and down in a direction along which the transmission device is arranged, here the two cooling pipelines arranged up and down may be partially arranged in the retarder, form a [structure and may be communicated with an external cooling system, the neutron generation part 20 increases temperature and generates heated due to accelerated irradiation of a high energy level, and the neutron generation part 20 is efficiently cooled by a cooling medium flowing through the cooling pipelines. In the embodiment as shown, the detection device 90 may be a temperature sensor, including a detection circuit arranged along the transmission device 10 and connected to a control end, and may be configured to detect a real-time temperature of the cooling device. In an alternative embodiment, the detection device may also be a charged particle sensor, configured to detect a charged particle beam before the first transmission part 11 reacts with the neutron generation part 20; or the detection device may be a vacuum pressure sensor, configured to detect a vacuum degree of the first transmission part 11; or the detection device may be a neutron detector, configured to detect neutrons generated after occurrence of a nuclear reaction. The detection device 90 includes, but is not limited to, an electronic sensor, a proximity sensor, a capacitive sensor, a transducer, or another form of sensor. When the first transmission part 11 is located at the first position L1, the cooling pipelines are communicated with pipelines of the external cooling system, to provide a state where cooling may be implemented; and the detection circuit and circuits of the control end are in a connected state, so that real-time monitoring and data transmission and feedback may be implemented. When the first transmission part 11 is located at the second position L2, the cooling pipelines are disconnected from pipelines of the external cooling system, and the detection circuit is disconnected from connection lines of the control end. The cooling device and the partially disconnected pipelines, and the partially disconnected circuit of the detection device are all arranged at the first transmission part, and move together with the first transmission part 11 to the second position to be recovered and replaced.

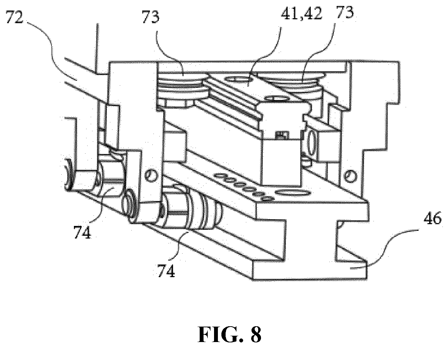

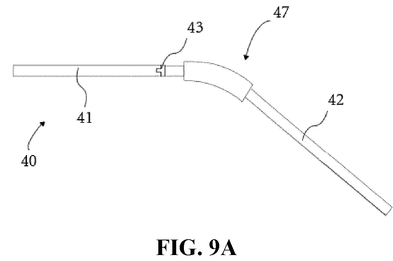

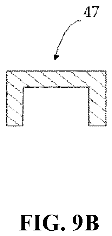

FIG. 8 shows that the driving frame 72 of the drive structure 70 includes at least one set of rollers, and in a preferred embodiment, the driving frame 72 includes at least two sets of rollers 73, 74. With reference to FIG. 1, FIG. 4, FIG. 6 and FIG. 8, the guidance device 40 further includes a rail base 46 and a plurality of segments of rails, preferably, the first rail 41 and the second rail 42 are laid on the rail base 46. Rolling planes of the two sets of rollers 73, 74 on the driving frame 72 are different, preferably, rolling directions of the two sets of rollers 73, 74 are also different, and it should be noted that rolling directions of rollers in a single set of rollers are all kept consistent. The roller set 73 and a rail segment above the base form a combination of the roller and a convex rail, the roller set 74 and the rail base 46 form a combination of the roller and a concave rail, the two combinations are in different modes, roll on different planes, and have rolling directions perpendicular to each other, which may reliably ensure high stability of the whole guidance operation in two dimensions, making a significant contribution to matching and adaptability of rails during subsequent switching of the rails, and even flexibility of selecting any one or two sets of rollers. In a preferred embodiment, the rail 422 structural segment where the second rail 42 may be flexibly rotated, is only provided with the rail base 46, so that during switching to the rail 422 segment, there is only guidance and cooperation of the roller set 74 with the concave rail base 46 of the rail 422 segment, guidance and cooperation of the roller set 73 with the convex rail of the rail 422 segment are released, to enter the third rail 44 inside the aligned radiation shielding device 50, and the third rail 44 is in linkage alignment with the concave rail base 46 of the rail 422 segment. Considering that weights of the first transmission part 11 and the neutron generation part 20 are large, when guidance and transmission are performed by using rollers to cooperate with a concave or convex rail in the embodiment, it is also necessary to comprehensively consider bearing capacity and wear resistance of the guidance device, in addition to considering metals which are not easily activated by neutrons and have a short half-life period. Therefore, selection of an aluminum alloy or aluminum-magnesium alloy material only may be unable to withstand large weights of the first transmission part 11 and the neutron generation part 20, as well as abrasion of rollers on guide rails. Therefore, the guidance device further considers selection of a wear-resistant material with large rigidity, and a surface of the material is coated with a coating film configured to prevent neutron activation and capable of resisting abrasion of rollers, to reduce probability of activation of the material and prolong service life of the rail. By performing cross-sectional stress analysis on a plurality of segments of the guide rail, it may be known that load and deformation borne by the cross section of the rail at a bent segment are much larger than those borne by the rail at a linear segment. The first rail 41 and a part of the second rail 42 are rails at linear segments, and the rest part of the second rail 42 needs to form a bent segment due to change of the guiding direction, therefore this segment has high requirements on rigidity and wear resistance of the material. According to the above research and analysis, in an alternative embodiment, considering that a linear guidance part located close to the beam shaping body 30 in the core reaction space R2 selects an aluminum alloy, a magnesium-aluminum alloy, and/or a material of which a surface is coated with a wear-resistant coating, a bent guidance part considers selection of stainless steel or another material with large rigidity, or considers a surface thereof to be coated with a coating film configured to prevent neutron activation and capable of resisting abrasion of rollers. Or, with reference to FIG. 9A and FIG. 9B, an anti-radiation protective cover 47 shaped to fit the guidance structure is made by using an anti-radiation material such as boron-containing PE, and/or a neutron or photon shielding material such as boron-containing resin, or the like. In an operation process of the radioactive irradiation system, the anti-radiation protective cover 47 is always covered on a surface of easy-to-be-activated material of the guidance device, and when radioactive consumables are replaced, the radioactive irradiation system stops operation at this time, so that the anti-radiation protective cover 47 may be removed, and the guide rail is used normally. According to requirements, a plurality of different materials are selected in segments on the rail of the guidance device, so that service life of the guidance device may be greatly prolonged, and usage reliability of the guidance device may be improved.

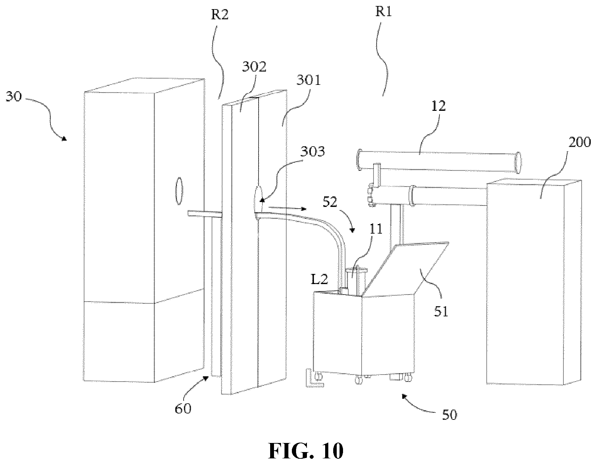

FIG. 10 is a schematic view of another embodiment of replacement of the neutron generation part, showing that the shielding facility 300 between the first space R1 and the second space R2 is in a closed state. The first space R1 is physically separated from the second space R2 when the shielding door 301 and/or the shielding door 302 are closed, and the guidance device 40 passes through the opening 303 of the shielding facility from the first space R1 into the second space R2. Preferably, in this state, the operator is located in the first space R1 and may safely operate in a condition of less radiation, to recover the first transmission part and neutron generation part. As in same operations in the embodiment of FIG. 3, the operator performs preparation operations of replacement operation, completes disassembling the second transmission part 12, and checking connection and position conditions of the first rail 41, the second rail 42 and the radiation shielding device 50. In the embodiment, preferably, the operator removes and cuts off pipelines corresponding to the cooling device 80 and the detection device 90 (referring to FIG. 7) on the first transmission part 11 by using his hands or other tools to pass through the opening 303 only; preferably, pipelines of other pneumatic devices (not shown) may also exist on the first transmission part 11 and are removed and cut off together, and then the first transmission part 11 with the neutron generation part is moved to a third position (not shown) close to the opening 303 of the shielding facility 300, and an end of the first transmission part 11 arranged with the neutron generation part is always kept to be located in the second space R2. Power from the driving device 70 (referring to FIG. 7), or a pulling force from the operator, or the like is used as a first power to be applied to the first transmission part 11, thus the first transmission part 11 with the neutron generation part generates a first movement with a small distance, and is separated and disconnected from the beam shaping body 30, and at this time, radiation value of the second space R2 is much higher than radiation value of the first space R1 due to exposure of the neutron generation part. It should be noted that when the neutron generation part is separated manually, the operator should be provided with appropriate protection and quickly evacuate to the safe area R. The power from the driving device 70 (referring to FIG. 7) or the pulling force from the operator is applied as a second power again, and under action of the second power, the first transmission part 11 performs a second movement with a relatively large distance on the first rail 41 in a first direction along which the transmission device 10 is arranged, to completely barrier-free pass through the opening 303 of the shielding facility 300 which is kept in a closed state, so as to move to the second rail 42 located in the first space R1, and under action of gravity or power of the rail, the first transmission part 11 continues to move to the second position L2 along the direction of the second rail 42. Shape of the opening 303 of the shielding facility 300 may be circular shape, square shape, or even irregular shape, or the like, and preferably, may be adapted to cross sections of the first transmission part 11 with the neutron generation part and of the guidance device, so that it may ensure that the first transmission part 11 with the neutron generation part may smoothly pass through the shielding facility 303 and continue to move to a recovery position after auxiliary pipelines related to water, electricity and gas are disconnected, while radiation leakage is reduced to the maximum extent. The opening 303 of the shielding facility 300 may also be provided with a shielding cover plate (not shown) made of a radiation shielding material, and during normal operation, the shielding cover plate does not shield the opening 303; and when the neutron generation part is replaced according to the above operations, the operator disassembles and disconnect the auxiliary pipelines, and moves the first transmission part 11 with the neutron generation part out, and then shields the opening 303 to the maximum extent by the movable shielding cover plate, to further reduce radiation leakage of the second space R2 to the first space R1.

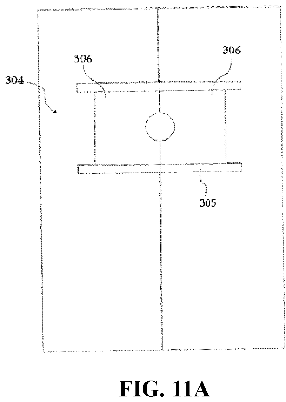

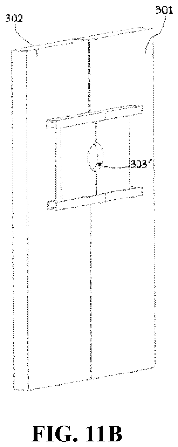

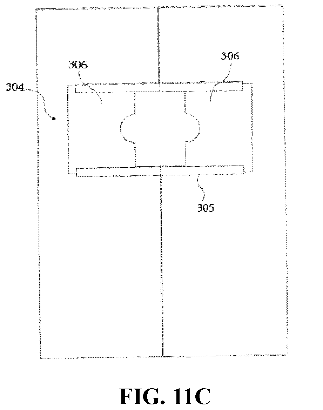

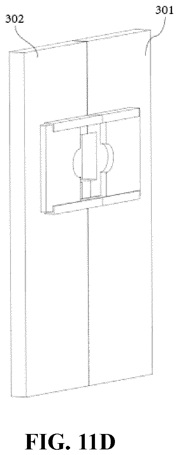

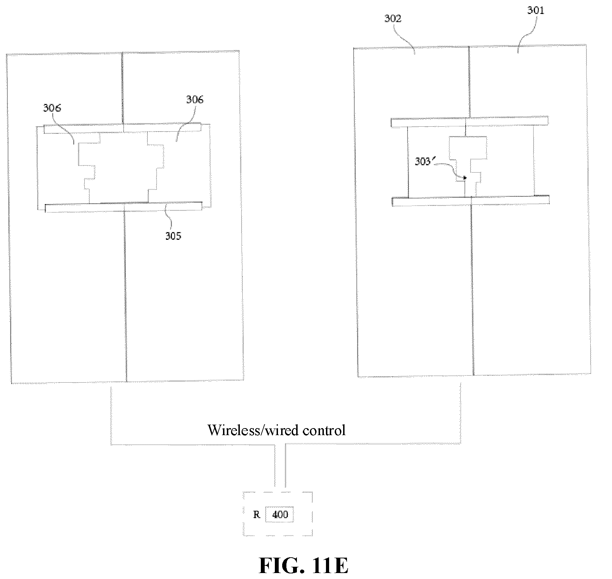

With reference to FIG. 11A to FIG. 11E, the shielding facility 300 between the first space R1 and the second space R2 is in a closed state. The first space R1 is physically separated from the second space R2 when the shielding door 301 and/or the shielding door 302 are closed, and the guidance device 40 passes through the opening 303 of the shielding facility from the first space R1 into the second space R2. With reference to FIG. 10, preferably, an auxiliary door 304 is arranged at the opening 303 of the shielding facility 300, similar to the above movable shielding cover plate. The auxiliary door 304 includes a fixed part 305 and a movable part 306. The movable part 306 is divided into left and right sections which are located on fixed parts 305 fixed on the shielding doors 301 and 302 respectively. When the movable part 306 is closed, there is at least one opening 303' in the middle. In the embodiment, shape of at least one opening 303' of the auxiliary door 304 is usually fitted with shapes of the first transmission part 11, the guidance device 40 and mating pipeline structures, a vacuum valve, or the like arranged at the opening 303', to reduce radiation leakage from the opening 303' when the shielding facility 300 and the auxiliary door 304 are closed to perform irradiation therapy of the neutron beam, and the shape includes, but is not limited to, oval shape, rhombus shape, square shape, or even an irregular shape, or the like (FIG. 11A to FIG. 11E). When recovery operation of the first transmission part and the neutron generation part is performed, only the auxiliary door 304 may be opened, the shielding facility 300 is kept closed, and after they are replaced by a new first transmission part and a new neutron generation part, the auxiliary door 304 is closed; or, when recovery operation of the first transmission part and the neutron generation part is performed, the shielding facility 300 is opened, and after they are replaced by a new first transmission part and a new neutron generation part, the shielding facility 300 and the auxiliary door 304 are closed. The auxiliary door 304 is made of a material with good shielding performance, and the movable member 306 of the auxiliary door 304 may be opened or closed along a path preset by the fixed member 305 when the shielding door 301 and the shielding door 302 are kept closed, such that design of such flexible, small and shape-fitted auxiliary door is beneficial to further reducing radiation amount during irradiation therapy of the neutron beam; furthermore, when the first transmission part or the guidance device is adjusted in shape or structure, only the auxiliary door needs to be adjusted or customized, without consuming manpower and material resources to modify the heavy and bulky shielding door, while energy of moving the bulky shielding door is also saved.

In an embodiment, a method for recovering the neutron generation part specifically includes the following operations S1 to S12.

In operation S1, the radiation shielding device is moved to the second space.

In operation S2, the first transmission part is kept in a vacuum state, and a second transmission part between the first transmission part and the third transmission part is removed.

In operation S3, auxiliary pipelines related to water, electricity and gas, such as the cooling pipeline and the detection circuit, are removed.

In operation S4, the radiation shielding device is pushed to position of a ground positioning member, to stop, lock and fix the radiation shielding device, and the openable member of the radiation shielding device is opened at this time.

In operation S5, the anti-radiation protective cover on the guidance device is removed, and the second guidance part is connected to and aligned with the third guidance part in the radiation shielding device.

In operation S6, the operator exits from the second space to a safe area.

In operation S7, a driving device switch on the master control panel is triggered, to drive the power structure to push out the driving frame where the first transmission part and the neutron generation part are installed, and move the driving frame to the radiation shielding device along the guidance device.

In operation S8, a control switch, for the openable member of the radiation shielding device, on the master control panel is triggered, so that the openable member is closed to prevent radiation leakage.

In operation S9, the anti-radiation protective cover is covered on the guidance device.

In operation S10, the ground positioning member is unlocked, and the radiation shielding device is pushed out of the second space.

In operation S11, a new neutron generation part is installed, to complete connection of the first transmission part, the second transmission part and the third transmission part.

In operation S12, auxiliary pipelines related to water, electricity and gas, such as the cooling pipeline and the detection circuit, are connected, and the transmission device is debugged.

Both embodiments of FIG. 5 and FIG. 6 are applicable to operations of replacing the neutron generation part in a state where the shielding facility is opened or closed; the structure detachably connected to the transmission device may also be fixed by a quick clamping structure, or may be a structure fixed by bolts and nuts, and a part of the transmission device is configured as a telescopic corrugated pipe, a folding pipe, a telescopic pipe, or the like. Furthermore, manners of driving the first transmission part to be disconnected from the beam shaping body and move, controlling the radiation shielding device to move to a preset position, opening and closing the openable shielding member, and docking and separating rails are not limited to movement caused by hands or gravity, and may also be an operator's short-range and remote operations and automatic control. The invention is defined by the appended claims.

## Claims

1. A neutron capture therapy system (100) comprising an accelerator (200) configured to generate a charged particle beam, a transmission device (10) configured to transmit the charged particle beam and comprising at least a first transmission part (11) and a second transmission part, a neutron generation part (20) configured to generate a neutron beam by reacting with the charged particle beam, a beam shaping body (30) configured to perform spectrum adjustment on the neutron beam, and a guidance device, wherein the first transmission part (11) is detachably connected to the second transmission part (12) and comprises a first position and a second position, the neutron generation part (20) is arranged on the first transmission part (11), and the guidance device (40) is capable of guiding the first transmission part (11) to move from the first position to the second position, and wherein when the first transmission part (11) is located at the first position, the neutron generation part (20) is accommodated in the beam shaping body (30) and is capable of reacting with the charged particle beam to generate neutrons; and when the first transmission part (11) is located at the second position, the neutron generation part (20) is separated from the beam shaping body to be recovered;
wherein the guidance device (40) comprises a first guidance part and a second guidance part, of which guiding directions are different from each other;
the neutron capture therapy system further comprises:
a movable radiation shielding device (50) configured to accommodate the first transmission part (11) and the neutron generation part (20) guided by the guidance device, and the guidance device (40) further comprising a third guidance part which is fixedly accommodated in the movable radiation shielding device;
a positioning system enabling the radiation shielding device (50) to move to a preset position to be locked, so that the second guidance part is docked with the third guidance part;
wherein the radiation shielding device (50) is made of a radiation shielding material and comprises an openable member, the radiation shielding device (50) forms an accommodation opening when the openable member is opened, wherein the first transmission part (11) of the transmission device (10) is configured to enter the radiation shielding device (50) from the accommodation opening, and then the neutron generation part(20) is configured to enter the radiation shielding device (50) from the accommodation opening, wherein the neutron generation part (20) is configured to enter the radiation shielding device (50) later than the first transmission part such that when recovery operation is completed, the neutron generation part (20) is closer to the accommodation opening 52 of the radiation shielding device (50); and wherein the radiation shielding device (50) forms a closed shielding space when the openable member is closed, to prevent radiation leakage of the neutron generation part; and
wherein the movable radiation shielding device (50) further comprises
a buffer device (54) capable of generating elastic deformation after the first transmission part (11) is in contact with the buffer device, to avoid damage to at least the first transmission part (11) or the radiation shielding device.

2. The neutron capture therapy system of claim 1, wherein in a plane where the first guidance part is located parallel to the ground, a projection of a connection line between a first end and a second end of the second guidance part forms a first angle with the first guidance part; and in a plane where the first guidance part is located perpendicular to the ground, a projection of the connection line between the first end and the second end of the second guidance part forms a second angle with the first guidance part.

3. The neutron capture therapy system of claim 1 or 2, wherein the second guidance part comprises at least two segments of guide rails, one of which is a fixed rail detachably connected to the first guidance part, and the other of which is movable rail rotatably connected to the fixed rail.

4. The neutron capture therapy system of claim 1, further comprising a driving device (70) which is capable of moving the first transmission part (11) arranged with the neutron generation part (20) out from the first position, and comprises a driving frame carrying the first transmission part (11) and the neutron generation part (20) and a power structure providing a driving force.

5. The neutron capture therapy system of claim 1, further comprising a control part capable of remotely controlling at least the driving device (70) or the movable radiation shielding device (50) through wired or wireless connection.

6. A method for recovering the neutron generation part of the neutron capture therapy system of claim 1, the neutron capture therapy system further comprising an auxiliary pipeline connected to the neutron generation part (20) or the first transmission part (11), the method for recovering the neutron generation part (20) comprising the following steps:
splitting the first transmission part (11) and the second transmission part (12) apart;
disconnecting the auxiliary pipeline from the neutron generation part;
applying a first power to the first transmission part (11) arranged with the neutron generation part, to separate the first transmission part (11) from the beam shaping body (30) along the guidance device (40) and from the first position; and
applying a second power to the first transmission part (11) arranged with the neutron generation part, to move the first transmission part (11) along the guidance device (40) again, until to a recovery position;
the neutron capture therapy system further comprises a shielding facility provided with an opening, and the first transmission part (11) passes through the opening in a state where the shielding facility is kept closed;
the neutron capture therapy system further comprises the radiation shielding device (50) configured to accommodate the neutron generation part (20) after recovery, and a first space and a second space separated by the shielding facility, an anti-radiation protective cover is arranged on the guidance device, the neutron generation part (20) is located in the first space, the second transmission part (12) is located in the second space, and the method for recovering the neutron generation part (20) further comprises:
before applying the first power to the first transmission part (11) arranged with the neutron generation part,
moving the radiation shielding device (50) to the second space and fixing the radiation shielding device (50) at a preset position;
opening the openable member of the radiation shielding device; and
removing the anti-radiation protective cover on the guidance device,
after applying the second power to the first transmission part (11) arranged with the neutron generation part,
moving the first transmission part (11) into the radiation shielding device (50) along the guidance device;
closing the openable member of the radiation shielding device;
covering the anti-radiation protective cover on the guidance device; and
pushing the radiation shielding device (50) out of the second space.

7. The method for recovering the neutron generation part of claim 6, being carried out through the opening in the state where the shielding facility is kept closed.

8. The method for recovering the neutron generation part of claim 6, wherein the shielding facility is provided with an auxiliary door, the auxiliary door at least partially shields the opening of the shielding facility and has an opening fitted with contours of the first transmission part (11), the guidance device (40) and the auxiliary pipeline arranged at the opening of the shielding facility when the auxiliary door is closed, and the method for recovering the neutron generation part (20) is carried out through the opening in a state where the shielding facility is kept closed and the auxiliary door is kept open.

## Patentansprüche

1. Ein Neutroneneinfangtherapiesystem (100), umfassend einen Beschleuniger (200), der dazu eingerichtet ist, einen geladenen Teilchenstrahl zu erzeugen, eine Übertragungsvorrichtung (10), die dazu eingerichtet ist, den geladenen Teilchenstrahl zu übertragen, und die mindestens einen ersten Übertragungsteil (11) und einen zweiten Übertragungsteil umfasst, einen Neutronenerzeugungsteil (20), der dazu eingerichtet ist, durch Reagieren mit dem geladenen Teilchenstrahl einen Neutronenstrahl zu erzeugen, einen Strahlformungskörper (30), der dazu eingerichtet ist, eine Spektrumanpassung an dem Neutronenstrahl durchzuführen, und eine Führungsvorrichtung, wobei der erste Übertragungsteil (11) lösbar mit dem zweiten Übertragungsteil (12) verbunden ist und eine erste Position und eine zweite Position aufweist, der Neutronenerzeugungsteil (20) an dem ersten Übertragungsteil (11) angeordnet ist und die Führungsvorrichtung (40) in der Lage ist, den ersten Übertragungsteil (11) derart zu führen, dass er sich von der ersten Position zu der zweiten Position bewegt, und wobei, wenn sich der erste Übertragungsteil (11) an der ersten Position befindet, der Neutronenerzeugungsteil (20) in dem Strahlformungskörper (30) aufgenommen ist und in der Lage ist, mit dem geladenen Teilchenstrahl zu reagieren, um Neutronen zu erzeugen; und wenn sich der erste Übertragungsteil (11) an der zweiten Position befindet, der Neutronenerzeugungsteil (20) von dem Strahlformungskörper getrennt ist, um wiederhergestellt zu werden;
wobei die Führungsvorrichtung (40) einen ersten Führungsteil und einen zweiten Führungsteil umfasst, deren Führungsrichtungen voneinander verschieden sind;
wobei das Neutroneneinfangtherapiesystem ferner umfasst:
eine bewegliche Strahlungsabschirmvorrichtung (50), die dazu eingerichtet ist, den ersten Übertragungsteil (11) und den Neutronenerzeugungsteil (20), die durch die Führungsvorrichtung geführt werden, aufzunehmen, wobei die Führungsvorrichtung (40) ferner einen dritten Führungsteil umfasst, der fest in der beweglichen Strahlungsabschirmvorrichtung aufgenommen ist;
ein Positionierungssystem, das es der Strahlungsabschirmvorrichtung (50) ermöglicht, sich in eine voreingestellte Position zu bewegen, um verriegelt zu werden, sodass der zweite Führungsteil an den dritten Führungsteil angedockt ist;
wobei die Strahlungsabschirmvorrichtung (50) aus einem Strahlungsabschirmmaterial hergestellt ist und ein öffenbares Element umfasst, wobei die Strahlungsabschirmvorrichtung (50) eine Aufnahmeöffnung bildet, wenn das öffenbare Element geöffnet ist, wobei der erste Übertragungsteil (11) der Übertragungsvorrichtung (10) dazu eingerichtet ist, von der Aufnahmeöffnung aus in die Strahlungsabschirmvorrichtung (50) einzutreten, und anschließend der Neutronenerzeugungsteil (20) dazu eingerichtet ist, von der Aufnahmeöffnung aus in die Strahlungsabschirmvorrichtung (50) einzutreten, wobei der Neutronenerzeugungsteil (20) dazu eingerichtet ist, später als der erste Übertragungsteil in die Strahlungsabschirmvorrichtung (50) einzutreten, sodass, wenn ein Wiederherstellungsvorgang abgeschlossen ist, der Neutronenerzeugungsteil (20) näher an der Aufnahmeöffnung 52 der Strahlungsabschirmvorrichtung (50) liegt;
und wobei die Strahlungsabschirmvorrichtung (50) einen geschlossenen Abschirmraum bildet, wenn das öffenbare Element geschlossen ist, um ein Austreten von Strahlung des Neutronenerzeugungsteils zu verhindern; und wobei die bewegliche Strahlungsabschirmvorrichtung (50) ferner eine Puffervorrichtung (54) umfasst, die in der Lage ist, eine elastische Verformung zu erzeugen, nachdem der erste Übertragungsteil (11) mit der Puffervorrichtung in Kontakt gelangt ist, um eine Beschädigung mindestens des ersten Übertragungsteils (11) oder der Strahlungsabschirmvorrichtung zu vermeiden.

2. Das Neutroneneinfangtherapiesystem nach Anspruch 1, wobei in einer Ebene, in der der erste Führungsteil parallel zum Boden angeordnet ist, eine Projektion einer Verbindungslinie zwischen einem ersten Ende und einem zweiten Ende des zweiten Führungsteils einen ersten Winkel mit dem ersten Führungsteil bildet; und in einer Ebene, in der der erste Führungsteil senkrecht zum Boden angeordnet ist, eine Projektion der Verbindungslinie zwischen dem ersten Ende und dem zweiten Ende des zweiten Führungsteils einen zweiten Winkel mit dem ersten Führungsteil bildet.

3. Das Neutroneneinfangtherapiesystem nach Anspruch 1 oder 2, wobei der zweite Führungsteil mindestens zwei Segmente von Führungsschienen umfasst, von denen eines eine feststehende Schiene ist, die lösbar mit dem ersten Führungsteil verbunden ist, und das andere eine bewegliche Schiene ist, die drehbar mit der feststehenden Schiene verbunden ist.

4. Das Neutroneneinfangtherapiesystem nach Anspruch 1, ferner umfassend eine Antriebsvorrichtung (70), die in der Lage ist, den ersten Übertragungsteil (11), der mit dem Neutronenerzeugungsteil (20) angeordnet ist, aus der ersten Position herauszubewegen, und einen Antriebsrahmen, der den ersten Übertragungsteil (11) und den Neutronenerzeugungsteil (20) trägt, sowie eine Kraftstruktur zum Bereitstellen einer Antriebskraft umfasst.

5. Das Neutroneneinfangtherapiesystem nach Anspruch 1, ferner umfassend einen Steuerteil, der in der Lage ist, mindestens die Antriebsvorrichtung (70) oder die bewegliche Strahlungsabschirmvorrichtung (50) über eine drahtgebundene oder drahtlose Verbindung aus der Ferne zu steuern.

6. Ein Verfahren zum Wiederherstellen des Neutronenerzeugungsteils des Neutroneneinfangtherapiesystems nach Anspruch 1, wobei das Neutroneneinfangtherapiesystem ferner eine Hilfsleitung umfasst, die mit dem Neutronenerzeugungsteil (20) oder dem ersten Übertragungsteil (11) verbunden ist, wobei das Verfahren zum Wiederherstellen des Neutronenerzeugungsteils (20) die folgenden Schritte umfasst:
Trennen des ersten Übertragungsteils (11) und des zweiten Übertragungsteils (12) voneinander;
Trennen der Hilfsleitung von dem Neutronenerzeugungsteil;
Anlegen einer ersten Kraft an den ersten Übertragungsteil (11), der mit dem Neutronenerzeugungsteil angeordnet ist, um den ersten Übertragungsteil (11) entlang der Führungsvorrichtung (40) von dem Strahlformungskörper (30) und aus der ersten Position zu trennen; und
Anlegen einer zweiten Kraft an den ersten Übertragungsteil (11), der mit dem Neutronenerzeugungsteil angeordnet ist, um den ersten Übertragungsteil (11) erneut entlang der Führungsvorrichtung (40) bis zu einer Wiederherstellungsposition zu bewegen;
wobei das Neutroneneinfangtherapiesystem ferner eine Abschirmeinrichtung umfasst, die mit einer Öffnung versehen ist, und der erste Übertragungsteil (11) in einem Zustand, in dem die Abschirmeinrichtung geschlossen gehalten wird, durch die Öffnung hindurchtritt;
wobei das Neutroneneinfangtherapiesystem ferner die Strahlungsabschirmvorrichtung (50), die dazu eingerichtet ist, den Neutronenerzeugungsteil (20) nach der Wiederherstellung aufzunehmen, und einen ersten Raum und einen zweiten Raum umfasst, die durch die Abschirmeinrichtung voneinander getrennt sind, wobei eine Strahlungsschutzabdeckung an der Führungsvorrichtung angeordnet ist, der Neutronenerzeugungsteil (20) in dem ersten Raum angeordnet ist, der zweite Übertragungsteil (12) in dem zweiten Raum angeordnet ist und das Verfahren zum Wiederherstellen des Neutronenerzeugungsteils (20) ferner umfasst:
vor dem Anlegen der ersten Kraft an den ersten Übertragungsteil (11), der mit dem Neutronenerzeugungsteil angeordnet ist,
Bewegen der Strahlungsabschirmvorrichtung (50) in den zweiten Raum und Fixieren der Strahlungsabschirmvorrichtung (50) an einer voreingestellten Position;
Öffnen des öffenbaren Elements der Strahlungsabschirmvorrichtung; und
Entfernen der Strahlungsschutzabdeckung von der Führungsvorrichtung,
nach dem Anlegen der zweiten Kraft an den ersten Übertragungsteil (11), der mit dem Neutronenerzeugungsteil angeordnet ist,
Bewegen des ersten Übertragungsteils (11) entlang der Führungsvorrichtung in die Strahlungsabschirmvorrichtung (50);
Schließen des öffenbaren Elements der Strahlungsabschirmvorrichtung;
Abdecken der Führungsvorrichtung mit der Strahlungsschutzabdeckung; und
Schieben der Strahlungsabschirmvorrichtung (50) aus dem zweiten Raum heraus.

7. Das Verfahren zum Wiederherstellen des Neutronenerzeugungsteils nach Anspruch 6, das durch die Öffnung in dem Zustand durchgeführt wird, in dem die Abschirmeinrichtung geschlossen gehalten wird.

8. Das Verfahren zum Wiederherstellen des Neutronenerzeugungsteils nach Anspruch 6, wobei die Abschirmeinrichtung mit einer Hilfstür versehen ist, die Hilfstür die Öffnung der Abschirmeinrichtung zumindest teilweise abschirmt und eine Öffnung aufweist, die an Konturen des ersten Übertragungsteils (11), der Führungsvorrichtung (40) und der an der Öffnung der Abschirmeinrichtung angeordneten Hilfsleitung angepasst ist, wenn die Hilfstür geschlossen ist, und das Verfahren zum Wiederherstellen des Neutronenerzeugungsteils (20) durch die Öffnung in einem Zustand durchgeführt wird, in dem die Abschirmeinrichtung geschlossen gehalten wird und die Hilfstür offen gehalten wird.

## Revendications

1. Un système de thérapie par capture neutronique (100) comprenant un accélérateur (200) configuré pour générer un faisceau de particules chargées, un dispositif de transmission (10) configuré pour transmettre le faisceau de particules chargées et comprenant au moins une première partie de transmission (11) et une deuxième partie de transmission, une partie de génération de neutrons (20) configurée pour générer un faisceau de neutrons en réagissant avec le faisceau de particules chargées, un corps de mise en forme de faisceau (30) configuré pour effectuer un ajustement de spectre sur le faisceau de neutrons, et un dispositif de guidage, dans lequel la première partie de transmission (11) est reliée de manière détachable à la deuxième partie de transmission (12) et comprend une première position et une deuxième position, la partie de génération de neutrons (20) est disposée sur la première partie de transmission (11), et le dispositif de guidage (40) est capable de guider la première partie de transmission (11) pour la déplacer de la première position à la deuxième position, et dans lequel, lorsque la première partie de transmission (11) est située dans la première position, la partie de génération de neutrons (20) est logée dans le corps de mise en forme de faisceau (30) et est capable de réagir avec le faisceau de particules chargées pour générer des neutrons ; et lorsque la première partie de transmission (11) est située dans la deuxième position, la partie de génération de neutrons (20) est séparée du corps de mise en forme de faisceau afin d'être récupérée ;
dans lequel le dispositif de guidage (40) comprend une première partie de guidage et une deuxième partie de guidage, dont les directions de guidage sont différentes l'une de l'autre ;
le système de thérapie par capture neutronique comprend en outre :
un dispositif mobile de blindage contre les rayonnements (50) configuré pour loger la première partie de transmission (11) et la partie de génération de neutrons (20) guidées par le dispositif de guidage, et le dispositif de guidage (40) comprenant en outre une troisième partie de guidage qui est logée de manière fixe dans le dispositif mobile de blindage contre les rayonnements ;
un système de positionnement permettant au dispositif de blindage contre les rayonnements (50) de se déplacer jusqu'à une position prédéfinie pour y être verrouillé, de sorte que la deuxième partie de guidage soit raccordée à la troisième partie de guidage ;
dans lequel le dispositif de blindage contre les rayonnements (50) est constitué d'un matériau de blindage contre les rayonnements et comprend un élément ouvrable, le dispositif de blindage contre les rayonnements (50) forme une ouverture de logement lorsque l'élément ouvrable est ouvert, dans lequel la première partie de transmission (11) du dispositif de transmission (10) est configurée pour entrer dans le dispositif de blindage contre les rayonnements (50) par l'ouverture de logement, puis la partie de génération de neutrons (20) est configurée pour entrer dans le dispositif de blindage contre les rayonnements (50) par l'ouverture de logement, dans lequel la partie de génération de neutrons (20) est configurée pour entrer dans le dispositif de blindage contre les rayonnements (50) après la première partie de transmission, de telle sorte que, lorsque l'opération de récupération est terminée, la partie de génération de neutrons (20) soit plus proche de l'ouverture de logement 52 du dispositif de blindage contre les rayonnements (50) ;
et dans lequel le dispositif de blindage contre les rayonnements (50) forme un espace de blindage fermé lorsque l'élément ouvrable est fermé, afin d'empêcher une fuite de rayonnement de la partie de génération de neutrons ; et dans lequel le dispositif mobile de blindage contre les rayonnements (50) comprend en outre un dispositif tampon (54) capable de générer une déformation élastique après que la première partie de transmission (11) est entrée en contact avec le dispositif tampon, afin d'éviter d'endommager au moins la première partie de transmission (11) ou le dispositif de blindage contre les rayonnements.

2. Le système de thérapie par capture neutronique selon la revendication 1, dans lequel, dans un plan dans lequel la première partie de guidage est située parallèlement au sol, une projection d'une ligne de liaison entre une première extrémité et une deuxième extrémité de la deuxième partie de guidage forme un premier angle avec la première partie de guidage ; et, dans un plan dans lequel la première partie de guidage est située perpendiculairement au sol, une projection de la ligne de liaison entre la première extrémité et la deuxième extrémité de la deuxième partie de guidage forme un deuxième angle avec la première partie de guidage.

3. Le système de thérapie par capture neutronique selon la revendication 1 ou 2, dans lequel la deuxième partie de guidage comprend au moins deux tronçons de rails de guidage, l'un étant un rail fixe relié de manière détachable à la première partie de guidage, et l'autre étant un rail mobile relié de manière rotative au rail fixe.

4. Le système de thérapie par capture neutronique selon la revendication 1, comprenant en outre un dispositif d'entraînement (70) qui est capable de déplacer, hors de la première position, la première partie de transmission (11) sur laquelle est disposée la partie de génération de neutrons (20), et comprend un châssis d'entraînement portant la première partie de transmission (11) et la partie de génération de neutrons (20) et une structure de puissance fournissant une force d'entraînement.

5. Le système de thérapie par capture neutronique selon la revendication 1, comprenant en outre une partie de commande capable de commander à distance au moins le dispositif d'entraînement (70) ou le dispositif mobile de blindage contre les rayonnements (50) par l'intermédiaire d'une connexion filaire ou sans fil.

6. Un procédé de récupération de la partie de génération de neutrons du système de thérapie par capture neutronique selon la revendication 1, le système de thérapie par capture neutronique comprenant en outre une conduite auxiliaire reliée à la partie de génération de neutrons (20) ou à la première partie de transmission (11), le procédé de récupération de la partie de génération de neutrons (20) comprenant les étapes suivantes :
séparer la première partie de transmission (11) et la deuxième partie de transmission (12) l'une de l'autre ;
déconnecter la conduite auxiliaire de la partie de génération de neutrons ;
appliquer une première force à la première partie de transmission (11) sur laquelle est disposée la partie de génération de neutrons, afin de séparer la première partie de transmission (11) du corps de mise en forme de faisceau (30) le long du dispositif de guidage (40) et de la déplacer hors de la première position ; et
appliquer une deuxième force à la première partie de transmission (11) sur laquelle est disposée la partie de génération de neutrons, afin de déplacer à nouveau la première partie de transmission (11) le long du dispositif de guidage (40), jusqu'à une position de récupération ;
le système de thérapie par capture neutronique comprend en outre une installation de blindage pourvue d'une ouverture, et la première partie de transmission (11) traverse l'ouverture dans un état dans lequel l'installation de blindage est maintenue fermée ;
le système de thérapie par capture neutronique comprend en outre le dispositif de blindage contre les rayonnements (50) configuré pour loger la partie de génération de neutrons (20) après récupération, ainsi qu'un premier espace et un deuxième espace séparés par l'installation de blindage, un capot de protection anti-rayonnement est disposé sur le dispositif de guidage, la partie de génération de neutrons (20) est située dans le premier espace, la deuxième partie de transmission (12) est située dans le deuxième espace, et le procédé de récupération de la partie de génération de neutrons (20) comprend en outre :
avant d'appliquer la première force à la première partie de transmission (11) sur laquelle est disposée la partie de génération de neutrons,
déplacer le dispositif de blindage contre les rayonnements (50) jusqu'au deuxième espace et fixer le dispositif de blindage contre les rayonnements (50) dans une position prédéfinie ;
ouvrir l'élément ouvrable du dispositif de blindage contre les rayonnements ; et
retirer le capot de protection anti-rayonnement sur le dispositif de guidage,
après avoir appliqué la deuxième force à la première partie de transmission (11) sur laquelle est disposée la partie de génération de neutrons,
déplacer la première partie de transmission (11) dans le dispositif de blindage contre les rayonnements (50) le long du dispositif de guidage ;
fermer l'élément ouvrable du dispositif de blindage contre les rayonnements ;
recouvrir le dispositif de guidage avec le capot de protection anti-rayonnement ; et
pousser le dispositif de blindage contre les rayonnements (50) hors du deuxième espace.

7. Le procédé de récupération de la partie de génération de neutrons selon la revendication 6, étant mis en œuvre à travers l'ouverture dans l'état dans lequel l'installation de blindage est maintenue fermée.

8. Le procédé de récupération de la partie de génération de neutrons selon la revendication 6, dans lequel l'installation de blindage est pourvue d'une porte auxiliaire, la porte auxiliaire masque au moins partiellement l'ouverture de l'installation de blindage et comporte une ouverture adaptée aux contours de la première partie de transmission (11), du dispositif de guidage (40) et de la conduite auxiliaire disposés au niveau de l'ouverture de l'installation de blindage lorsque la porte auxiliaire est fermée, et le procédé de récupération de la partie de génération de neutrons (20) est mis en œuvre à travers l'ouverture dans un état dans lequel l'installation de blindage est maintenue fermée et la porte auxiliaire est maintenue ouverte.
